# EUROPÄISCHE PATENTANMELDUNG

(11) **EP 1 987 852 A2**
(43) Veröffentlichungstag der Anmeldung: **05.11.2008**
(21) Anmeldenummer: 08153933.0
(22) Anmeldetag: 02.04.2008
(51) Int. Cl.: A61M 5/44

(54) **Temperiertasche**

(30) Priorität: 04.05.2007 DE 102007021406
(71) Anmelder: Litterst,, Herr Werner-Alfons, 77654 Offenburg (DE)
(72) Erfinder: Litterst,, Herr Werner-Alfons, 77654 Offenburg (DE)
(74) Vertreter: Wolf, Eckhard

(57) **Zusammenfassung**

Die Erfindung betrifft eine Temperiervorrichtung für Flüssigkeiten, insbesondere für Infusionslösungen, Blut, Blutplasma, Kontrastmittel oder Nährlösungen, mit aus einem flexiblen Material gebildeten Vorder- und Rückwänden (10, 12), die zusammen eine Tasche mit einer Einführöffnung (22) für einen Flüssigkeitsbehälter, insbesondere einen Infusionsbeutel bilden, wobei die Vorder- und/oder Rückwand mit einem Heizelement (14) versehen ist.

## Beschreibung

Die Erfindung betrifft eine Temperiervorrichtung für Flüssigkeiten, insbesondere für Infusionslösungen, Blut, Blutplasma, Kontrastmittel oder Nährlösungen.

Bekannt sind derartige Vorrichtungen für Glasflaschen. Diese werden jedoch immer häufiger von flexiblen Beuteln ersetzt, die mit den bekannten Vorrichtungen nicht temperiert werden können. Bei Infusionen ist es wichtig, dass das Infusat nicht wesentlich von der Körpertemperatur von etwa 37 °C abweicht. Ein zu großer Temperaturunterschied, der meistens in Richtung zu niedriger Temperaturen tendiert, kann zu Gerinnungen führen, die Embolien auslösen können. Eine erheblich von der Körpertemperatur abweichende Temperatur eines Infusats wird darüber hinaus von dem Infusionsempfänger als unangenehm empfunden.

Ausgehend hiervon besteht die Aufgabe der vorliegenden Erfindung darin, eine Temperiervorrichtung bereitzustellen, mit der Infusionsbeutel genau und sicher temperiert werden können.

Zur Lösung dieser Aufgabe wird die in dem Patentanspruch 1 angegebene Merkmalskombination vorgeschlagen. Vorteilhafte Ausgestaltungen und Weiterbildungen der Erfindung ergeben sich aus den abhängigen Ansprüchen.

Erfindungsgemäß weist die Temperiervorrichtung für Flüssigkeiten, insbesondere für Infusionslösungen, Blut, Blutplasma, Kontrastmittel oder Nährlösungen, aus einem flexiblen Material gebildete Vorder- und Rückwände auf, die zusammen eine Tasche mit einer Einführöffnung für einen Infusionsbeutel bilden, wobei die Vorder- und/oder Rückwand mit einem Heizelement versehen ist. Durch die flexible Ausbildung der Tasche kann sich diese gut an einen mit Flüssigkeit gefüllten Beutel anschmiegen, auch wenn dieser sich im Laufe einer Infusion stetig leert, und ermöglicht so eine gute Wärmeübertragung von der Heizung auf die Flüssigkeit.

Bevorzugt sind die Vorder- und Rückwand aus einem zweilagigen Kunststoffmaterial gebildet, wobei dass das Heizelement vorteilhaft nach Art einer Scheibenheizung bei einem PKW mäanderförmig zwischen den zwei Lagen der Vorder- und/oder Rückwand verläuft. Ein bevorzugtes Kunststoffmaterial ist ein vorzugsweise glasfaserverstärkter Silikonkunststoff. Ein derartiges Material weist trotz eines gegenüber anderen Kunststoffen hohen Preises eine Reihe von Vorteilen auf. Beispielsweise lässt es sich gut reinigen, ist also unter hygienischen Gesichtspunkten vorteilhaft. Es ist mechanisch stark belastbar und kann deswegen über einen langen Zeitraum wiederverwendet werden. Weiterhin weist es gute thermische Isolationseigenschaften auf, ist elektrisch nicht leitend und in dem betreffenden Temperaturbereich schmelzsicher.

Die Einführöffnung kann in der Benutzungsstellung der Vorrichtung an ihrer Oberseite angeordnet und durch mindestens eine Lasche abdeckbar sein. Eine oben angeordnete Einführöffnung erleichtert das Einführen eines Infusionsbeutels, da ein solcher in der Regel in seinem unteren Bereich eine Anschlussröhre für einen Infusionsschlauch umfasst, der durch eine den unteren Bereich der Vorder- und Rückwände durchbrechende Öffnung hindurchtritt. Ein Einführen des Beutels beispielsweise von der Seite her würde das Durchführen der Anschlussröhre durch die dafür vorgesehene Öffnung erschweren. Die Verschlusslasche verhindert Wärmeverluste über die Einführöffnung und verringert somit die erforderliche Heizenergiezufuhr. Sie schließt zweckmäßig an die Rückwand an und ist im oberen Bereich der Vorderwand mittels eines Magnet-, Klett- oder Druckknopfverschlusses fixierbar.

Weiterhin kann die Vorrichtung in vorteilhafter Weiterbildung der Erfindung einen an der Rückwand angeordneten, diese nach oben überragenden Stiel mit einer Öse zur Aufhängung der Vorrichtung an einem Infusionsständer aufweisen.

Um die elektrischen Heizelemente mit Strom zu versorgen weist die Vorrichtung in bevorzugter Ausgestaltung der Erfindung einen vorzugsweise an der Vorderwand angeordneten Anschlussblock auf, der eine vorzugsweise als Leuchtdiode ausgebildete Betriebsanzeigeeinrichtung aufweisen kann. Ein Netzteil für die Stromversorgung der Heizelemente ist vorteilhaft entfernt von der Tasche angeordnet und über ein Kabel mit dieser verbunden.

Weiterhin ist es von Vorteil, wenn mindestens ein Temperaturfühler zur Überwachung der Temperatur der Flüssigkeit vorgesehen ist. Bevorzugt weist die Vorrichtung zwei Temperaturfühler auf, von denen der eine den Istwert der Temperatur misst und der andere als Grenzwertüberwacher ein Steuersignal zum Abschalten der Heizung bei Erreichen einer vorgegebenen Maximaltemperatur erzeugt. Auf diese Weise kann die Heizung derart geregelt werden, dass die Abweichung der Flüssigkeitstemperatur von einem vorbestimmten Sollwert nicht mehr als 1 °C beträgt.

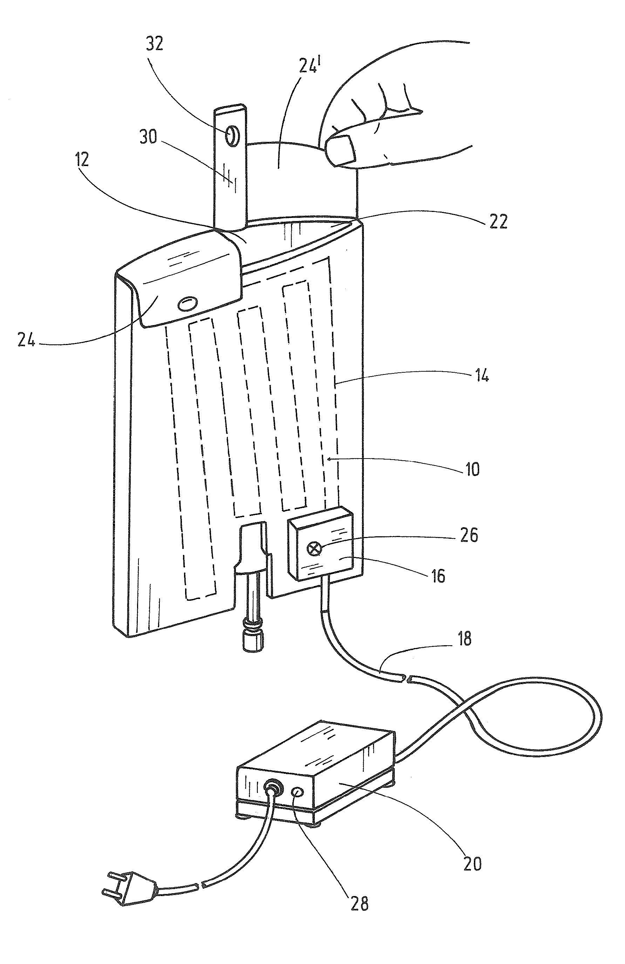

Im Folgenden wird die Erfindung anhand eines in der Zeichnung in schematischer Weise dargestellten Ausführungsbeispiels näher erläutert. Es zeigt
- Fig. 1: eine perspektivische Ansicht einer Vorrichtung zum Temperieren von Flüssigkeiten.

Die in der Zeichnung dargestellte Vorrichtung besteht im Wesentlichen aus einer Vorderwand 10 und einer Rückwand 12, die an ihren seitlichen und unteren Rändern miteinander verbunden sind und eine Tasche zur Aufnahme von Beuteln mit Flüssigkeiten wie Blut, Blutplasma, Kontrastmittel, Nährlösungen und dergleichen, die einem Patienten als Infusion zugeleitet werden, bilden. Die Wände 10 und 12 bestehen jeweils aus zwei Lagen eines glasfaserverstärkten Silikonkunststoffs, zwischen denen ein Heizelement 14 angeordnet ist. Die Heizelemente sind an einen Anschlussblock 16 angeschlossen, der über ein Kabel 18 mit einem geregelten Netzteil 20 verbunden ist. In dem Netzteil 20 befindet sich auch die Regelelektronik für die Heizung. Die Heizleistung beträgt bei einer Auslegung für Standardinfusionsbeutel mit einem Volumen von 100 ml, 200 ml und 500 ml etwa 30 W. Es gibt jedoch auch sehr große Infusionsbeutel mit einem Volumen von 5.000 ml. Hierfür ist eine entsprechend größere Temperiertasche mit einer entsprechend höheren Heizleistung erforderlich. Schließlich gibt es Großbehältnisse mit einem Inhalt von 200 bis 250 I, die temperiert werden müssen. Die Tasche ist für solche Anwendungen als Überstülptasche ausgebildet und weist eine Heizleistung im kW-Bereich auf.

Der Innenraum der Tasche ist über eine oben gebildete Einführöffnung 22 zugänglich, die nach dem Einführen eines Beutels von zwei Laschen 24, 24' abgedeckt werden kann, um einen Wärmeverlust über die Öffnung zu vermeiden. Die Temperaturmessung bzw. -überwachung erfolgt mittels zweier nicht näher dargestellter Temperaturfühler, die in dem Anschlussblock 16 enthalten sind. Der erste Temperaturfühler misst den Istwert der Beuteltemperatur, während der zweite Temperaturfühler bei Erreichen einer vorgegebenen Maximaltemperatur ein Signal erzeugt, dass zum Abschalten der Heizung führt. Wird über den ersten Temperaturfühler festgestellt, dass eine vorgegebene Mindesttemperatur unterschritten wird, so wird die Heizung wieder eingeschaltet. Auf diese Weise lässt sich die Temperatur in einem sehr engen Bereich von einigen zehntel Grad regeln.

Der ordnungsgemäße Betrieb der Vorrichtung wird durch eine Leuchtdiode 26 am Anschlussblock 16 angezeigt. Eine eventuelle Störung wird durch eine weitere Leuchtdiode 28 am Netzteil 20 angezeigt. Zur Aufhängung der Tasche an einem Infusionsständer ist an der Rückwand 12 ein Stiel 30 mit einer Öse 32 angeordnet.

## Patentansprüche

1. Temperiervorrichtung für Flüssigkeiten, insbesondere für Infusionslösungen, Blut, Blutplasma, Kontrastmittel oder Nährlösungen, mit aus einem flexiblen Material gebildeten Vorder- und Rückwänden (10, 12), die zusammen eine Tasche mit einer Einführöffnung (22) für einen Flüssigkeitsbehälter, insbesondere einen Infusionsbeutel bilden, wobei die Vorder- und/oder Rückwand mit einem Heizelement (14) versehen ist.

2. Vorrichtung nach Anspruch 1, **dadurch gekennzeichnet, dass** die Vorder- und Rückwand (10, 12) aus einem zweilagigen Kunststoffmaterial gebildet sind.

3. Vorrichtung nach Anspruch 2, **dadurch gekennzeichnet, dass** das Heizelement (14) mäanderförmig zwischen den zwei Lagen der Vorder- und/oder Rückwand verläuft.

4. Vorrichtung nach Anspruch 2 oder 3, **dadurch gekennzeichnet, dass** das Kunststoffmaterial ein vorzugsweise glasfaserverstärkter Silikonkunststoff ist.

5. Vorrichtung nach einem der Ansprüche 1 bis 4, **dadurch gekennzeichnet, dass** die Einführöffnung (22) in der Benutzungsstellung der Vorrichtung an ihrer Oberseite angeordnet ist und durch mindestens eine Lasche (24, 24') abdeckbar ist.

6. Vorrichtung nach Anspruch 5, **dadurch gekennzeichnet, dass** die mindestens eine Lasche (24, 24') an die Rückwand (12) anschließt und im oberen Bereich der Vorderwand (10) mittels eines Magnet-, Klett- oder Druckknopfverschlusses fixierbar ist.

7. Vorrichtung nach einem der Ansprüche 1 bis 6, **gekennzeichnet durch** einen an der Rückwand (12) angeordneten, diese nach oben überragenden Stiel (30) mit einer Öse (32) zur Aufhängung der Vorrichtung an einem Infusionsständer.

8. Vorrichtung nach einem der Ansprüche 1 bis 7, **gekennzeichnet durch** einen vorzugsweise an der Vorderwand (10) angeordneten Anschlussblock (16) für die Stromversorgung des mindestens einen Heizelements (14).

9. Vorrichtung nach Anspruch 8, **dadurch gekennzeichnet, dass** der Anschlussblock (16) eine vorzugsweise als Leuchtdiode ausgebildete Betriebsanzeigeeinrichtung (26) aufweist.

10. Vorrichtung nach einem der Ansprüche 1 bis 9, **gekennzeichnet durch** ein entfernt von der Tasche angeordnetes Netzteil (20) für die Stromversorgung der Heizung.

11. Vorrichtung nach einem der Ansprüche 1 bis 10, **gekennzeichnet durch** mindestens einen Temperaturfühler zur Überwachung der Temperatur der Flüssigkeit.

12. Vorrichtung nach Anspruch 11, **gekennzeichnet durch** zwei Temperaturfühler, von denen der eine den Istwert der Temperatur misst und der andere als Grenzwertüberwacher ein Steuersignal zum Abschalten der Heizung bei Erreichen einer vorgegebenen Maximaltemperatur erzeugt.

13. Vorrichtung nach Anspruch 12, **dadurch gekennzeichnet, dass** die Heizung derart geregelt wird, dass die Abweichung der Flüssigkeitstemperatur von einem vorbestimmten Sollwert nicht mehr als 1 °C beträgt.

14. Vorrichtung nach einem der Ansprüche 1 bis 13, **gekennzeichnet durch** eine den unteren Bereich der Vorder- und Rückwände durchbrechende Öffnung für den Durchtritt einer an einem Infusionsbeutel angeordneten Anschlussröhre für einen Infusionsschlauch
